# EUROPÄISCHE PATENTSCHRIFT

(11) **EP 0 606 072 B1**
(45) Veröffentlichungstag und Bekanntmachung des Hinweises auf die Patenterteilung: **10.06.1998**
(21) Anmeldenummer: 94100078.8
(22) Anmeldetag: 05.01.1994
(51) Int. Cl.: C07C 1/207, C07C 17/00, C07C 41/01, C07C 29/141, C07C 33/20, C07C 33/46, C07C 43/164, C07C 43/17, C07C 51/347, C07C 51/367, C07C 51/487

(54) **Selektive katalytische Hydrierung von aromatischen Aldehyden**
Selective catalytic hydrogenation of aromatic aldehydes
Procédé pour l'hydrogénation sélective de aldehydes aromatiques

(30) Priorität: 08.01.1993 DE 4300297
(43) Veröffentlichungstag der Anmeldung: 13.07.1994
(73) Patentinhaber: Degussa Aktiengesellschaft, 60311 Frankfurt (DE)
(72) Erfinder: Bankmann, Martin, Dr., D-63571 Gelnhausen (DE); Brand, Reinhold, Dr., D-63452 Hanau (DE); Freund, Andreas, Dr., D-63776 Mömbris (DE); Tacke, Thomas, Dr., D-63579 Freigericht (DE)

(56) Entgegenhaltungen:
- EP-A- 0 265 137
- EP-A- 0 535 565
- GB-A- 899 009
- GB-A- 1 159 967
- CATALYSIS TODAY Bd. 14, Nr. 2 , 4. Mai 1992 , AMSTERDAM,NL Seiten 225 - 242 M BANKMANN ET AL 'FORMING OF HIGH SURFACE AREA TIO2 TO CATALYST SUPPORTS'

## Beschreibung

Die Erfindung betrifft ein Verfahren zur selektiven, katalytischen Hydrierung der Carbonylgruppe von 4-Carboxybenzaldehyd in einem Gemisch aus 4-Carboxylbenzaldehyd/Terephthalsäure in Wasser bei 100 bis 300°C und einem Wassertoffpartialdruck von 5 bis 50 bar an einem Palladium -Trägerkatalysator.

Die selektive katalytische Hydrierung von aromatischen Aldehyden zu Benzylalkoholen bzw. den als Hydrogenolysefolgeprodukten entstehenden Toluol-Verbindungen ist für die Herstellung organischtechnischer Zwischenprodukte im Bereich der Feinchemie von großer Wichtigkeit.

Sie ermöglicht einen technisch einfach zu realisierenden Zugang zu diesen Produktklassen und stellt z. B. einen potenten alternativen Syntheseweg für die Herstellung substituierter Benzylalkohole dar, die ansonsten nach organisch-präparativen Verfahren durch gekreuzte Canizarro-Reaktion eines aromatischen Aldehyds mit Formaldehyd zugänglich sind.

In "Katalytische Hydrierungen" von F. Zymalkowski (Ferdinand Enke Verlag, Stuttgart 1965; Seiten 103 - 105) werden als geeignete Hydrierkatalysatoren Palladium auf Aktivkohle (Pd/C) und Nlckelkatalysatoren genannt. In "Forming of High Surface Area TiO₂ to Catalysts Supports" von Bankmann et al. in Catalysis Today 14 (1992) 225 - 242 werden Edelmetall/Titanoxid-Katalysatoren (EM/TiO₂) für die Hydrierung von aromatischen Aldehyden beschrieben.

Herausragende technische Bedeutung hat die selektive Hydrierung von aromatischen Aldehyden im Bereich der Reinigung von Terephthalsäure oder auch von p-Hydroxymethylbenzoesäure erlangt.

Terephthalsäure wird durch radikalkatalysierte Flüssigphasenoxidation von p-Xylol in Essigsäure mittels Co-Mn-Acetat-Katalysatoren und Bromaktivierung hergestellt (K. Weissermel, H.J. Arpe in "Industrieller Organische Chemie"; Verlag Chemie, Weinheim 1988, Selten 415 - 424). Die so hergestellte Terephthalsäure enthält als Verunreinigung 4-Carboxybenzaldehyd, das das spätere Polymerisationsverfahren zu Polyestern stört. Im Reinigungsschritt des Prozesses wird deshalb die Rohterephthalsäure in Wasser bei 275 bis 300° C unter Druck gelöst und in Gegenwart von Pd/C- oder Rh/C-Katalysatoren zu p-Hydroxymethylbenzoesäure und p-Methylbenzoesäure hydriert, bzw. zu Benzoesäure decarbonyllert.

Die genannten Reaktionsprodukte lassen sich durch fraktionierte Kristallisation aufgrund ihrer im Vergleich zu 4-Carboxybenzaldehyd und insbesondere zu Terephthalsäure größeren Wasserlöslichkeit aus der Rohterephthalsäure entfernen.

Man erhält Terephthalsäure in sogenannter faserreiner Qualität mit einer Reinheit von 99.99 %, die dann in Form von Dimethylterephthalat mit Diolkomponenten (bevorzugterweise Ethylenglykol) zu Polyethylenterephthalat (PET) umgesetzt wird. Hauptverwendung dieser Polyester ist der Fasersektor.

Für die Hydrierung von 4-Carboxybenzaldehyd bei der TA-Reinigung werden nach der EP 0 265 137 bevorzugt 0,5 % Pd/C (Kokosnußkohlel-Katalysatoren eingesetzt.

Als weitere Katalysatoren für die TA-Reinigung sind aus der DE 27 09 525 bimetallische Pt-Rh/C- bzw. Pd-Gruppe VIII Metall/C-Mischkatalysatoren, aus der USP 880 007 Ni/Kieselgur-Katalysatoren und aus der USP 4,743,577 Pd, Pt, Rh, Ni, Ru, Co bzw. deren Mischungen auf Metallträgern aus der Gruppe Ti, Zr, W, Cr, Ni oder Legierungen dieser Metalle bekannt.

Ein Problem beim Einsatz der beschriebenen EM/C-Katalysatoren ist der mechanische Abrleb der schwarzen Kohle-Festbettkatalysatoren, der als "black dots" zu nicht spezifikationsgerechter Faserqualität der gereinigten Terephthalsäure führt.

Aufgabe der vorliegenden Erfindung ist es, ein Verfahren zur selektiven katalytischen Hydrierung der Carbonylgruppe von 4-Carboxylbenzaldehyd in einem Gemisch aus 4-Carboxylbenzaldehyd/Terephthalsäure in Wasser, welches die genannten Nachteile bei der Reinigung von Terephthalsäure vermeidet.

Diese Aufgabe wird durch ein Verfahren gemäß Oberbegriff des einzigen Anspruchs gelöst, welches dadurch gekennzeichnet ist, daß als Tragermaterial ein Titanoxid verwendet wird, welches durch Flammenhydrolyse hergestellt ist, eine Anatas/Rutil-Mischphase mit einem Rutilgehalt von 25-100 Gew.-% und einen pH-Wert zwischen 3 und 7 aufweist und das Palladium mit einer Schalendicke von kleiner als 100 µm auf dem Träger vorliegt.

Es wurde gefunden, daß pyrogenes, durch Flammenhydrolyse aus Titantetrachlorid hergestelltes Titanoxid für die Herstellung der Titanoxidträger eingesetzt werden kann.

Pyrogenes Titanoxid besteht aus einer Mischphase aus 75 % Anatas und 25 % Rutil, welche gegebenenfalls durch entsprechende Kalzination der daraus geformten Trägerkörper vollständig in die Rutilmodifikation überführt werden kann. Durch diese Phasenumwandlung wird die Acidität des Titanoxids deutlich verringert. Die Acidität von Titanoxidpulvern wird nach ASTM D 3830-80 durch Messen des pH-Wertes einer 4%igen wäßrigen Suspension bestimmt. Pyrogenes Titanoxid aus 75 % Anatas und 25 % Rutilweist pH-Werte zwischen 3 und 5 und kalziniertes pyrogenes Titanoxid aus 100 % Rutil pH-Werte zwischen 5 und 7 auf.

Vorteilhaft sind Titanoxidqualitäten mit spezifischen BET-Oberflächen zwischen 1 und 200 m²/g, die gemäß DIN 66132 mit Stickstoff bestimmt werden.

Die Trägerkörper werden bevorzugt durch Extrusion hergestellt. Hierzu wird pyrogenes Titanoxidpulver unter Zusatz von 0,5 bis 3 Gew.-% Tylose, 0,2 bis 3 Gew.-% Milchsäure und Wasser auf einem Feuchtegehalt von 28 bis 38 % angefeuchtet und bei Raumtemperatur in einem Knetaggregat intensiv verknetet. Die resultierende plastische Knetmasse wird anschließend zu zylinderförmigen Trägerkörpern mit Durchmessern von 1 bis 5 mm und Längen von 5 bis 10 mm extrudiert.

Die so erhaltenen Grünkörper werden schonend bei 70° C für die Dauer von 24 Stunden getrocknet und dann zur Entfernung der organischen Zuschlagstoffe kalziniert.

Durch Wahl der Kalzinationstemperatur im Bereich von 400 bis 1000° C und der Kalzinationsdauer zwischen 1 und 5 Stunden kann das Anatas/Rutil-Verhältnis in den extrudierten Trägerkörpern definiert eingestellt werden.

Zur Erhaltung der ursprünglichen Phasenstruktur und der spezifischen Oberfläche der Ausgangsmaterialien wird bevorzugt bei 400° C nur 1 Stunde lang kalziniert.

Durch Erhöhung der Temperatur auf 700° C und fünfstündiges Kalzinieren kann die Phasenstruktur des pyrogenen Titanoxids vollständig in Rutil umgewandelt werden. Dadurch verringert sich die spezifische Oberfläche auf etwa 10 m²/g.

Die auf diese Weise hergestellten Trägerkörper besitzenein Gesamtporenvolumen zwischen 0,15 und 0,5 ml/g, welches sich aus Mesoporen mit Durchmessern zwischen 2 und 30 nm und Makroporen mit Durchmessern größer als 30 nm zusammensetzt. Mikroporen mit Durchmessern kleiner als 2 nm treten nicht auf.

Das Gesamtporenvolumen wird rechnerisch aus der Summe der Mikro-, Meso- und Makroporen bestimmt. Dabei erfolgt die Bestimmung der Mikro- und Mesoporen durch Aufnahme einer N₂-Isotherme und deren Auswertung nach BET, de Boer und Barret, Joyner, Halenda. Die Bestimmung der Makroporen erfolgt durch das QuecksilberEinpreßverfahren.

Die Trägerkörper weisen hervorragende mechanische Eigenschaften auf. Die Bruchfestigkeit, bestimmt mit dem Bruchfestigkeitstester der Firma Erweka, Typ TBH 28, liegt je nach Kalzinationstemperatur und Dauer zwischen 40 und 80 N. Diese Bruchfestigkeiten gewährleisten, daß der Abrleb der Trägerkörper gering ist und die dadurch verursachten Verunreinigungen der Reaktionsprodukte vernachlässigbar sind.

Diese mechanischen Eigenschaften der Trägerkörper werden ohne den Zusatz von anorganischen Bindemitteln wie zum Beispiel Bentonit erzielt, was wiederum zu einer hohen chemischen Beständigkeit der Trägerkörper führt. Die gute chemische Beständigkeit ist bei der Reinigung von Terephthalsäure von besonderem Vorteil, der hierbei in korrosiven, sauren Reaktionsmedien gearbeitet wird. Hierbei liegt die Terephthalsäure gewöhnlich im Überschuß zu den verunreinigenden aromatischen Aldehyden vor.

Die katalytisch aktiven Metallkomponenten werden durch bekannte Imprägnierverfahren in die kalzinierten Trägerkörper eingebracht. Geeignete Imprägnierverfahren sind zum Beispiel die Tauchimprägnierung oder die Sprühimprägnierung unter Berücksichtigung des Porenvolumen-Imprägnlerverfahrens. Bevorzugt wird die Sprüh-Imprägnierung eingesetzt.

Beim Porenvolumen-Imprägnierverfahren werden die löslichen Vorstufen der Metallkomponenten in einer Wassermenge gelöst, welche etwa 95 % des Wasseraufnahmevermögens der Trägerkörper entspricht, wobei der Metallgehalt der Lösung entsprechend dem gewünschten Metallgehalt der fertigen Katalysatoren eingestellt wird. Die Lösung wird dann mittels einer Sprühdüse, vorzugsweise einer Ultraschall-Sprühdüse, auf die in einer rotierenden Trommel umgewälzten Trägerkörper gesprüht.

Anschließend werden die so erhaltenen Katalysator-Grünkörper bei 120° C 15 Minuten lang im Wirbeltrockner getrocknet und danach, wie oben beschrieben, kalziniert.

Die Aktivierung der Katalysatoren wird in einem Formiergasstrom (95 % N₂, 5 % H₂) bei 280° C für die Dauer von einer Stunde vorgenommen. Dabei werden die Vorstufen der katalytisch aktiven Metallkomponenten reduziert.

Erfindungsgemäß wird als katalytisch aktive Metallkomponente Palladium eingesetzt. Geeignete Vorstufen für das Imprägnierverfahren sind wasserlösliche Verbindungen von Palladium, wie zum Beispiel Halogenide, Nitrate, Amminkomplexe und Nitrito- oder Nitrokomplexe.

Die Verwendung von Chloriden oder Nitraten ist nicht gleichwertig, sondern führt zu katalytischen Systemen mit unterschiedlichen Aktivitäten und Selektivitäten. Bei der Kalzinierung der imprägnierten Trägerkörper werden die Palladium verbindungen zersetzt. Während Nitrat unter reduktiven Bedingungen aus dem Katalysator restlos entfernt wird, verbleiben Reste von Chlor auf dem Katalysatorträger und verleihen ihm saure Eigenschaften (J.A.R. von Veen; Z. f. Phys. Chem. 162 (1989) 215-229).

Das sich einstellende Profil des Palladiums über den Querschnitt der Katalysatorkörper hängt von der Art des Edelmetallsalzes, dem pH-Wert der Imprägnierlösung und der Dauer zwischen Imprägnieren und Trocknen der Trägerkörper ab. Durchimprägnierte Katalysatoren werden zum Beispiel bei Verwendung von H₂PdCl₄, H₂PtCl₆ oder RhCl₃ und Ansäuerung der Imprägnierlösungen auf einen pH-Wert zwischen 0 und 1 erhalten. Schalenprofile mit Schalendicken von bis zu 100 µm werden dagegen erhalten, wenn die Imprägnierlösung mit Natronlauge auf einen pH-Wert von 5 eingestellt wird oder wenn an Stelle der Chloridverbindungen Nitratverbindungen des Palladiums verwendet werden.

Das Imprägnierprofil kann nach Aktivieren der Katalysatoren in einfacher Weise durch Anfertigen eines Querschliffes optisch vermessen werden. Die reduzierten Edelmetalle färben die ansonsten weißen Katalysatorkörper an den imprägnierten Stellen schwarz.

Sehr hohe Aktivitäten und Selektivitäten für die Bildung der aromatischen Alkohole werden erhalten, wenn Palladium in Form seiner Nitratverbindungen in den Träger mit einer schalenförmigen Verteilung eingebracht wird. Dieser Katalysator eignet sich gut für die Reinigung von p-Hydroxymethylbenzoesäure von dem als Nebenprodukt bei der Synthese entstehendem 4-Carboxybenzaldehyd durch selektive Hydrierung zu p-Hydroxymethylbenzoesäure. In diesem Fall darf die im Überschuß vorhandene p-Hydroxymethylbenzoesäure selbst nicht weiter hydriert werden.

Die Erfindung wird nun anhand einiger Beispiele näher erläutert. '

Zur Durchführung des erfindungsgemäßen Verfahrens wurden die in Tabelle 1 aufgeführten Edelmetall/TitanoxidKatalysatoren (EM/TiO₂) K1 bis K3 hergestellt. Als Vergleichskatalysator diente ein Palladium/Aktivkohle-Katalysator VK1 (Pd/C) wie er in den bekannten Hydrierverfahren eingesetzt wird sowie ein Pd/TiO₂-Katalysator VK2 auf 100% Anatas.
Die Katalysatoren K1 bis K3 sowie die Vergleichskatalysatoren wurden als Schalenkatalysatoren mit einer Schalendicke von unter 100 µm hergestellt. Dabei diente für die Katalysatoren VK1, K1 und K2 H₂PdCl₄ als Vorstufe für Palladium. Die Ausbildung der Schale wurde durch Einstellen des pH-Wertes der Imprägnierlösung auf 5 durch Zugabe von Natronlauge erreicht. Die Schale des Katalysators K2 wurde durch Verwendung von Palladium(II)-nitrat, Pd(NO₃)₂ erzielt.

Zur Charakterisierung der Metalldispersion wurde die CO-Adsorption an den Metallkristalliten ermittelt.

Die CO-Adsorption war auf dem reinen Anatas-Katalysatorkörper (Katalysator VK2) mit 0,1 CO/g Katalysator trotz der großen spezifischen Oberfläche dieses Materials von 92 m²/g am niedrigsten. Die beste Metalldispersion lag auf den Katalysatorkörpern mit einem Phasenverhältnis Anatas/Rutil von 75/25 vor. Aber auch Katalysator K2 auf reinem Rutil mit einer spezifischen Oberfläche von nur 18 m²/g zeigte noch eine überraschend hohe CO-Adsorption.

Das erfindungsgemäße Verfahren wurde beispielhaft in einem Autoklaven von einem Liter Fassungsvermögen durchgeführt. Der Autoklav war mit einem Katalysatorkorb, Begasungsrührer und einem Probenahmesystem ausgerüstet. Es wurden die folgenden Reaktionsbedingungen eingehalten:

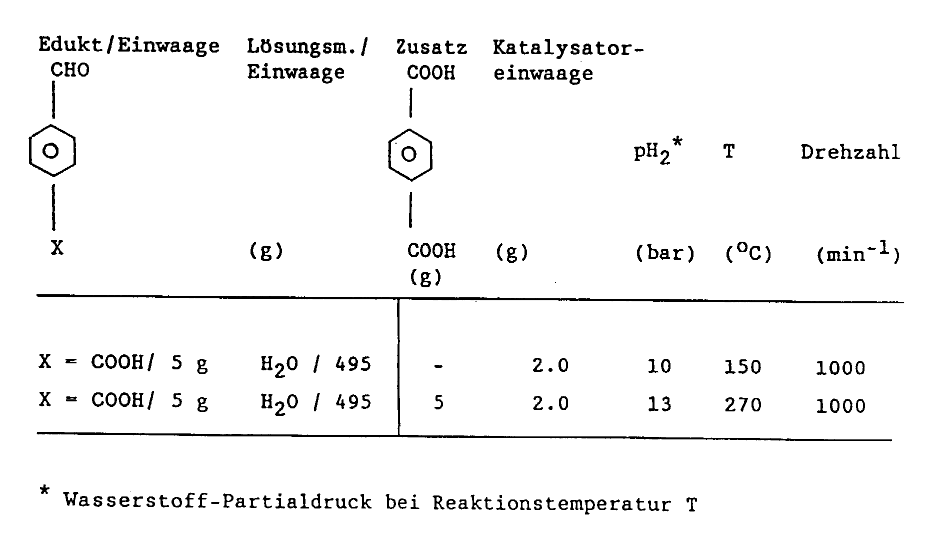

Der Katalysator wurde in den Katalysatorkorb gegeben und mit Raschigringen zur Fixierung eingebettet. In die Reaktionskammer des Autoklaven wurde dann das Edukt und anschließend das Lösungsmittel eingefüllt. Unter leichtem Rühren bei 200 min⁻¹ wurde auf Reaktionstemperatur aufgeheizt. Nach Erreichen der Solltemperatur wurde die Rührerdrehzahl auf 1000 min⁻¹ erhöht und 15 Minuten gerührt. Nachfolgend wurde eine Blindprobe gezogen und dann der Wasserstoff aufgedrückt.

Zur Verfolgung des Reaktionsverlaufs wurden jeweils nach 0,17 Stunden (≙ 10 Minuten), 1 Stunde, 2 Stunden und 4 Stunden weitere Proben von jeweils 2,5 ml gezogen und in 3,5 molarer NH₄OH-Lösung (5,0 ml) gelöst.

Bei der Hydrierung von 4-Carboxybenzaldehyd wurde der Gehalt der Proben an noch nicht umgesetztem Edukt mit HPLC (High Pressure Liquid Chromatography) ermittelt. Dazu wurden die Proben mit vollentsalztem Wasser weiter auf das 500-fache verdünnt.

Im Falle der Hydrierung von 4-Chlorbenzaldehyd bzw. p-Toluylaldehyd wurde die Umsetzung dieser Edukte mit GC Gas Chromatography) verfolgt.

### Beispiel 1

In Tabelle 2 sind die Aktivitäts- und Selektlvltätswerte des 0,5 % Pd/C-Vergleichskatalysators (VK1) auf Basis eines Kokosnußschalenaktivat-Trägermaterials und des 0,5 % Pd/TiO₂Katalysators (K1) für die Umsetzung einer 1 %igen wäßrigen Lösung von 4-Carboxybenzaldehyd (CBA) im 1 l Rührautoklav bei 150° C und 10 bar Wasserstoffdruck aufgelistet.

Der zeitabhängige Hydrierverlauf zeigt, daß der Pd/TiO₂-Katalysator K1 hochaktiv ist und CBA mit der gleichen sehr hohen Geschwindigkeit umsetzt wie der Pd/C-Vergleichskatalysator VK1.

Der Pd/C-Katalysator VK1 liefert mit hoher Selektivität p-Hydroxymethylbenzoesäure (HMBA), während mit dem Pd/TiO₂-Katalysator K1 in Abhängigkeit von der Reaktionsdauer bei kurzer Reaktionszeit die Alkoholkomponente HMBA oder bei längerer Reaktionszeit das Hydrogenolyse-Folgeprodukt p-Methylbenzoesäure (MBA) entsteht.

### Beispiel 2

In Tabelle 3 sind die Hydrierergebnisse an CBA bei Verwendung von Pd/TiO₂-Katalysatoren mit verschiedenen Anatas/Rutil-Phasenverhältnissen im TiO₂-Träger aufgeführt.

Durch Einsatz von Pd/TiO₂-Schalenkatalysatoren auf Basis von TiO₂-Trägern mit verschiedenen Anatas/Rutll-Phasen-Verhältnissen wird die Selektivität bei der Hydrierung von CBA wesentlich beeinflußt.

So wird mit dem Pd/TiO₂-Katalysator VK2 auf Basis eines reinen Anatas-Trägers bei einem CBA-Umsatz von 89 % mit einer Selektivität von über 95 % HMBA gebildet. Pd/TiO₂-Katalysator K1 mit einem 25 %igen Rutil-Anteil im Träger-System liefert hingegen bei gleichen Reaktionsbedingungen 55 % des Hydrogenolyseprodukts p-Methylbenzoesäure. Mit dem Pd/TiO₂-Katalysator K2 auf Basis eines reinen Rutil-Trägers wiederum entsteht neben dem Hauptprodukt HMBA zu 14,7 % das Decarbonylierungsprodukt Benzoesäure.

### Beispiel 3

Tabelle 4 zeigt den Einfluß der Edelmetallvorstufe bei der Herstellung des Pd/TiO₂-Katalysatorsystems auf die Selektivität.

Der mit H₂PdCl₄ imprägnierte Pd/TiO₂-Katalysator K1 führt bei nahezu quantitativem Umsatz zu einem Gemisch aus dem Hydrierprodukt HMBA und dem Hydrogenolyse-Folgeprodukt MBA. Beim Einsatz eines mit Pd(NO₃)₂ imprägnierten Pd/TiO₂-Katalysators K3 entsteht hingegen mit sehr hoher Selektivität die Alkoholkomponente HMBA. Die HMBA-Bildungsrate ist dabei auf dem gleichen Niveau wie beim Pd/C-Vergleichskatalysator VK1.

### Beispiel 4

In Tabelle 5 sind die Ergebnisse der CBA-Hydrierung in Gegenwart von Terephthalsäure bei 270°C mit den Pd/TiO₂-Katalysatoren VK2, K1, K2, K3 und dem Pd/C-Vergleichskatalysator VK1 zusammengestellt.

Alle getesteten Pd/TiO₂-Katalysatoren sind in Gegenwart von Terephthalsäure unter den drastischen Reaktionsbedingungen von 270° C mechanisch und chemisch stabil.

Auflösung, mechanische Ablösung oder Zerfall der Katalysatorformkörper treten nicht auf.

Dies ist darauf zurückzuführen, daß die Titanoxid-Katalysatoren eine sehr hohe mechanische Festigkeit aufweisen und die zugrunde liegenden TiO₂-Trägermaterialien säureresistent sind und ohne Zusatz von anorganischen Bindemitteln, die sich in organischen Säuren lösen könnten, verformt worden sind.

Überraschend ist, daß das EM/TiO₂-Katalysatorsystem bei den hohen Reaktionstemperaturen die Terephthalsäure überhaupt nicht hydriert, sondern hochselektiv den 4-Carboxybenzaldehyd abbaut.

Die Terephthalsäure-Konzentration bleibt bei allen Umsetzungen über den gesamten Hydrierverlauf konstant.

Die Pd/TiO₂-Katalysatoren zeigen in Abhängigkeit von dem Anatas/Rutil-Phasenverhältnis des zugrunde liegenden TiO₂-Trägers in Gegenwart von Terephthalsäure tendenziell das gleiche Selektivitätsmuster der HMBA/MBA-Produktverteilung wie bei der CBA-Hydrierung bei 150° C.

Die zusätzlich auftretende Decarbonylierung zu Benzoesäure ist auf die hohe Reaktionstemperatur zurückzuführen. Mit dem Pd/C-Vergleichskatalysator VK1 wird als Hauptprodukt Benzoesäure mit einer Selektivität von 55,6 % gebildet.

Der Pd/TiO₂-Katalysator VK2 auf Basis von reinem Anatas liefert bei vergleichsweise geringem CBA-Umsatz mit 71,4 % die höchste HMBA-Selektivität. Mit dem Pd/TiO₂-Katalysator K1 auf Basis des Anatas/Rutil-Milschträgers wird bevorzugt das Hydrogenolyseprodukt MBA gebildet.

Der vergleichbare über die Nitrat-Imprägniermethode hergestellte Pd/TiO₂-Katalysator K3 führt bei einer etwa gleich hohen CBA-Umsatzrate von über 90 % zu einem deutlichen Überschuß an HMBA gegenüber MBA.

Mit dem Rutil-, Pd/TiO₂-Katalysator K2 wird die Hydrogenolysereaktion zu p-Methylbenzoesäure weitgehend unterdrückt.

Neben dem Decarbonylierungsprodukt Benzoesäure entsteht als Haupt-Hydrierprodukt mit einer Selektivität von 61,5 % p-Hydroxymethylbenzoesäure.

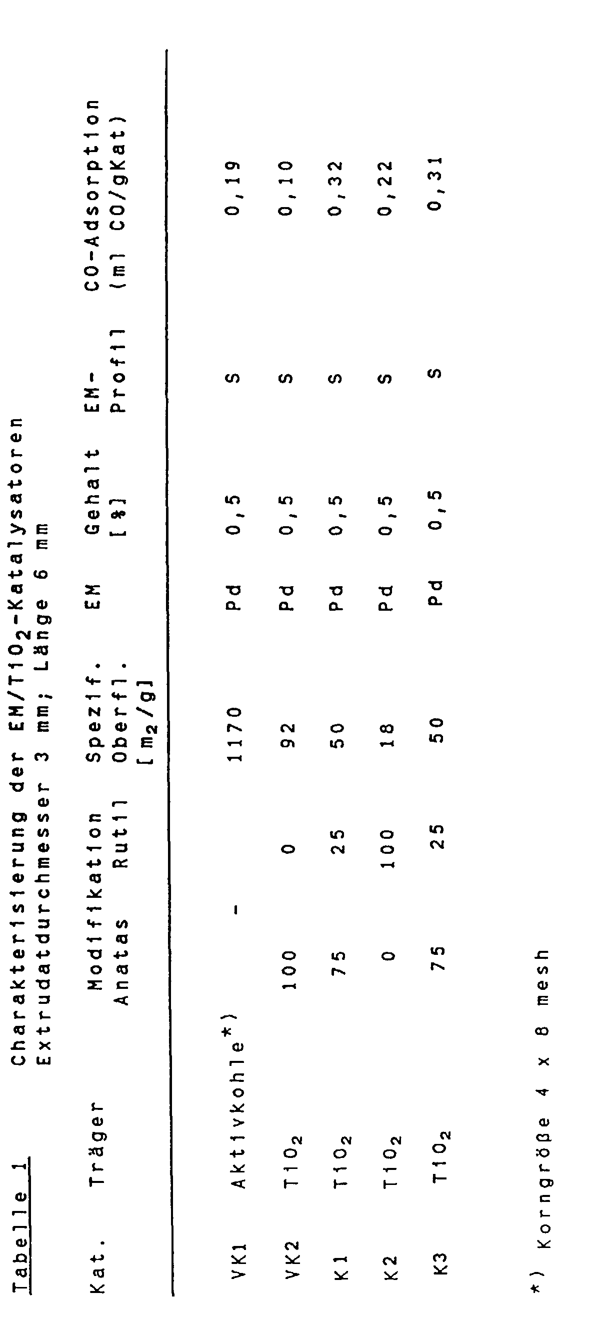

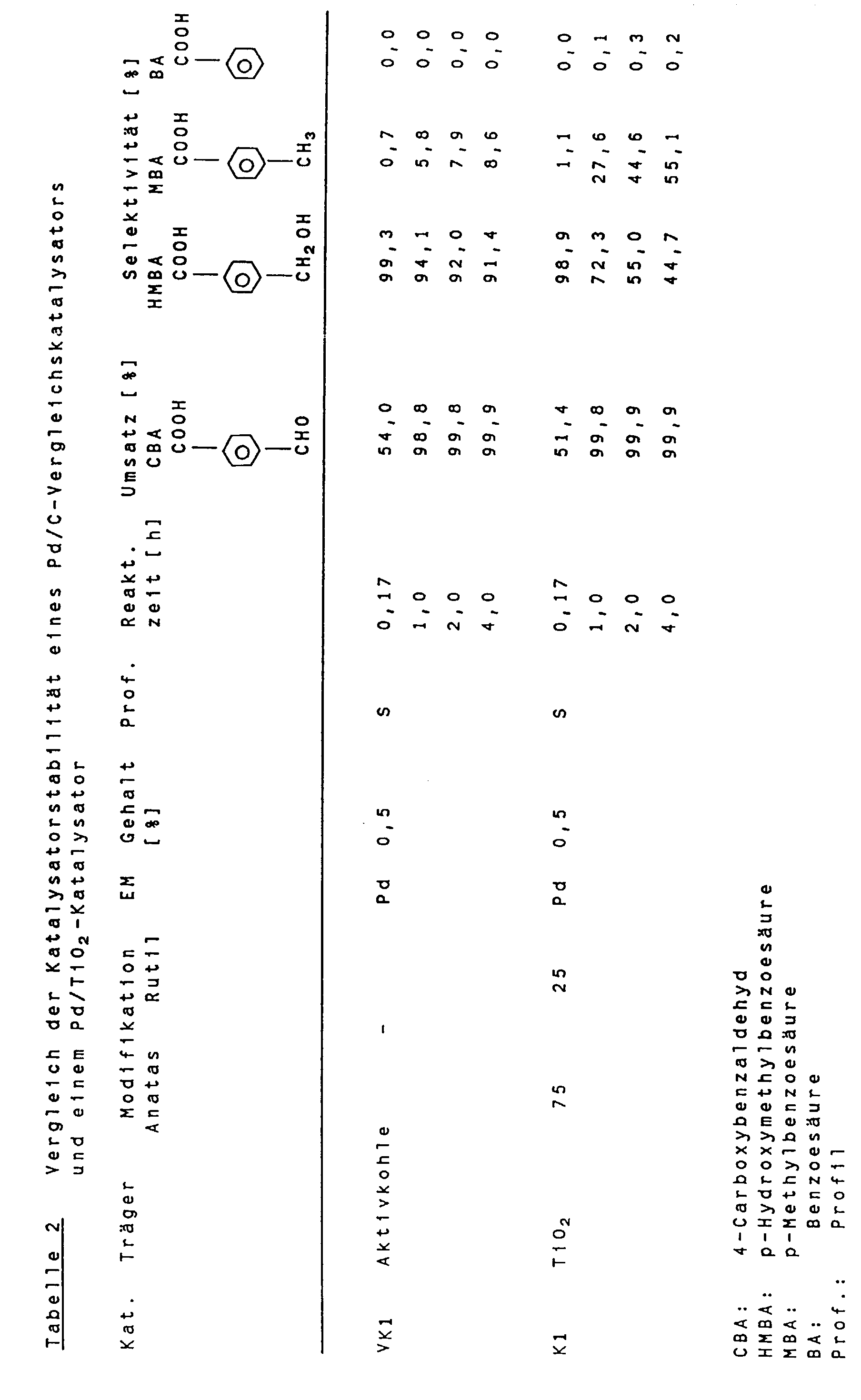

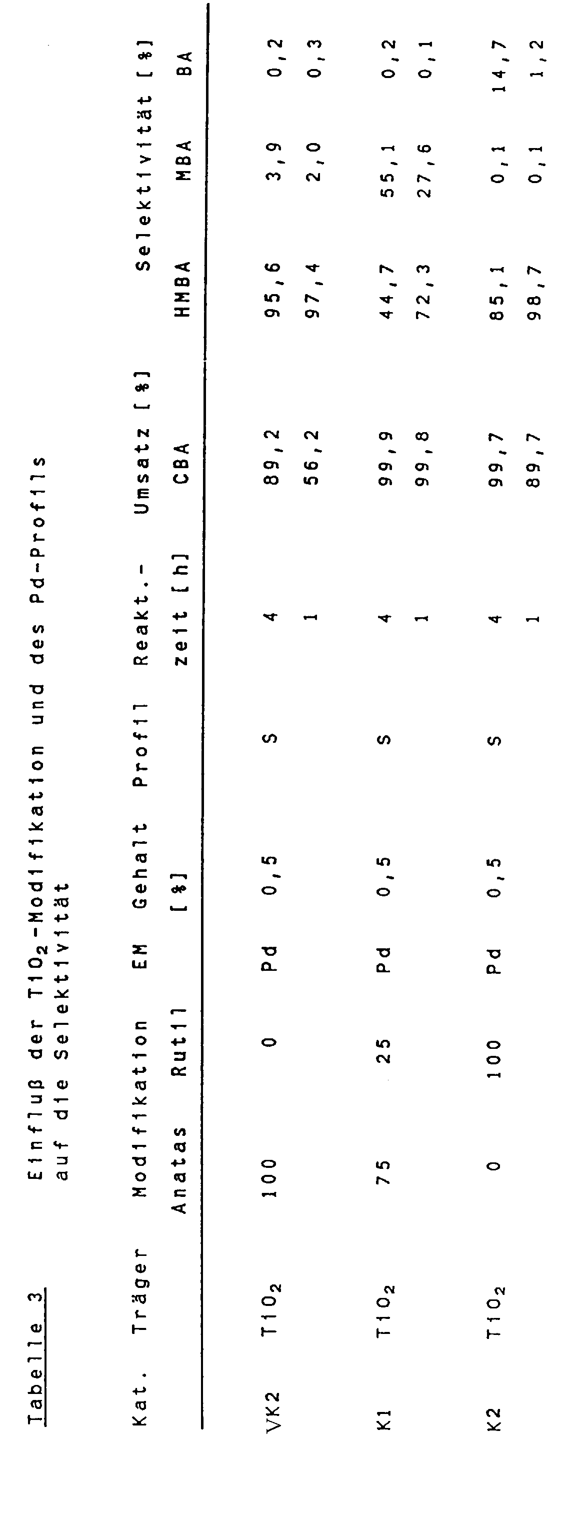

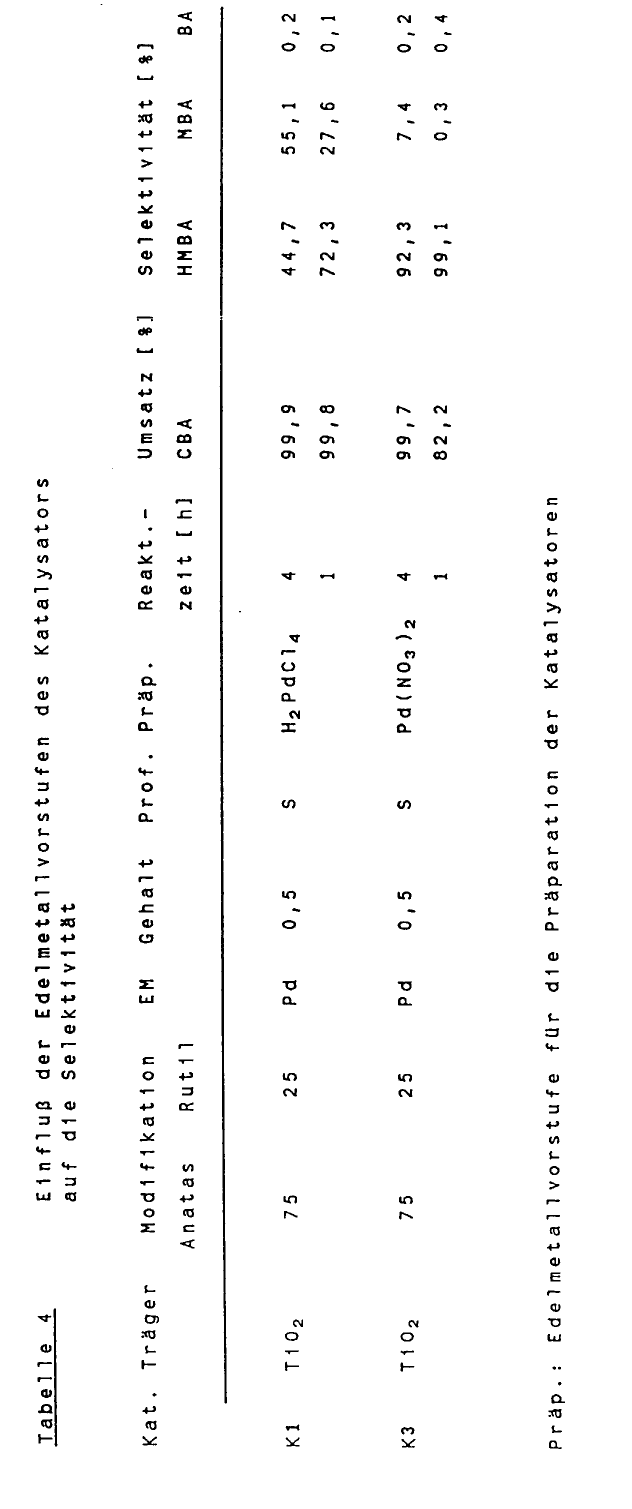

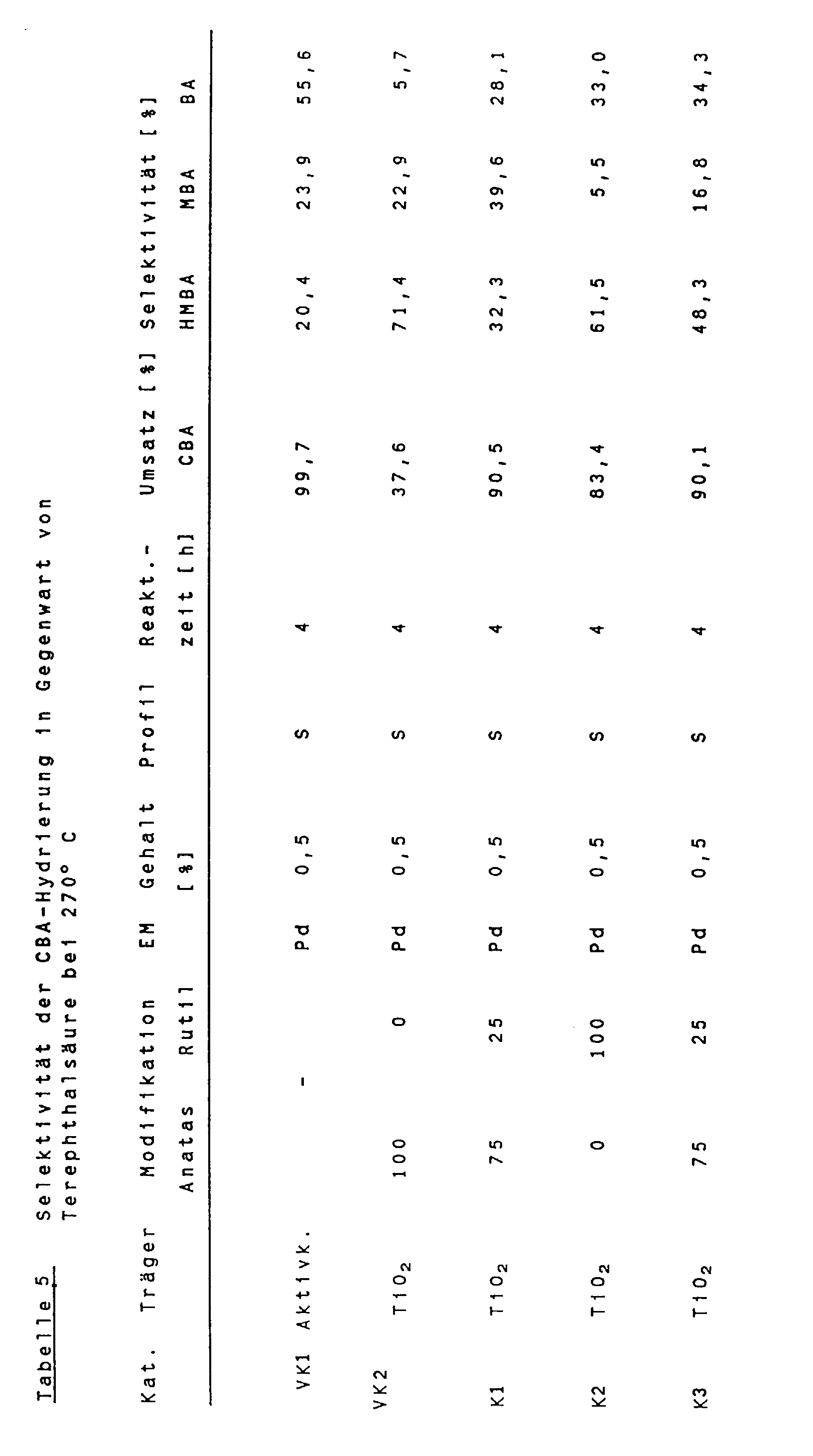

## Patentansprüche

1. Verfahren zur selektiven Hydrierung der Carbonylgruppe von 4-Carboxybenzaldehyd in einem Gemisch aus 4-Carboxybenzaldehyd/Terephthalsäure in Wasser bei 100 bis 300°C und einem Wasserstoffpartialdruck von 5 bis 50 bar an einem geformten Palladium-Trägerkatalysator,
**dadurch gekennzeichnet,**
daß als Trägermaterial ein Titanoxid verwendet wird, welches eine Anatas/Rutil-Mischphase mit einem Rutilgehalt von 25 - 100 Gew.-% und einen pH-Wert von 3 bis 7 aufweist und daß das Palladium mit einer Schalendicke von kleiner 100 µm auf dem Träger vorliegt.

## Claims

1. Process for the selective hydrogenation of the carbonyl group of 4-carboxybenzaldehyde in a mixture of 4-carboxybenzaldehyde/terephthalic acid in water at 100 to 300°C and a hydrogen partial pressure of 5 to 50 bar on a shaped, supported palladium catalyst,
characterised in that
the support material used is a titanium oxide which has an anatase/rutile mixed phase with a rutile content of 25-100 wt.% and a pH value of 3 to 7 and that the palladium is present on the support at a shell thickness of less than 100 µm.

## Revendications

1. Procédé d'hydrogénation sélective du groupe carbonyle du 4-carboxybenzaldéhyde dans un mélange de 4-carboxybenzaldéhyde/acide téréphtalique dans l'eau entre 100 et 300°C et à une pression partielle d'hydrogène de 5 à 50 bars sur un catalyseur support de palladium façonné,
caractérisé en ce qu'
on utilise comme matériau support un oxyde de titane qui présente une phase mixte anatase/rutile avec une teneur en rutile de 25-100 % en poids et un pH de 3 à 7, et en ce que le palladium se présente sur le support avec une épaisseur de couche inférieure à 100 µm.
